# EUROPÄISCHE PATENTSCHRIFT

(11) **EP 0 655 204 B1**
(45) Veröffentlichungstag und Bekanntmachung des Hinweises auf die Patenterteilung: **22.01.1997**
(21) Anmeldenummer: 94113103.9
(22) Anmeldetag: 23.08.1994
(51) Int. Cl.: A23L 1/015, A23L 1/308, A61K 35/10, C07G 17/00, A21D 2/08, A01N 61/00

(54) **Huminate enthaltende Diätetika**
Dietetic products containing huminates
Produits diététiques contenant des huminates

(30) Priorität: 27.11.1993 DE 4340485
(43) Veröffentlichungstag der Anmeldung: 31.05.1995
(73) Patentinhaber: Rütgers Aktiengesellschaft, 60326 Frankfurt (DE)
(72) Erfinder: Kühnert, Manfred, Prof., D-04299 Leipzig (DE); Haase, Anton Franz, Dr., D-64367 Mühltal (DE); Kleffner, Hans Werner, Dr., D-67271 Battenberg (DE)
(74) Vertreter: Patentanwälte Zellentin & Partner

(56) Entgegenhaltungen:
- EP-A- 0 214 362
- EP-A- 0 281 678
- EP-A- 0 281 679
- EP-A- 0 313 718
- EP-A- 0 537 427
- EP-A- 0 537 429
- EP-A- 0 537 430
- DE-A- 3 826 963
- DATABASE WPI Section Ch, Week 8713, Derwent Publications Ltd., London, GB; Class D16, AN 87-088650 & HU-A-40 817 (VITALTEK BORASZATI) 27. Februar 1987
- DATABASE WPI Section Ch, Week 7631, Derwent Publications Ltd., London, GB; Class C03, AN 76-59132X & JP-B-51 022 050 (MITSUI TOATSU CHEM. INC.) 8. Juli 1976

## Beschreibung

Die Erfindung betrifft die Verwendung von synthetisch hergestellten Huminaten zur Herstellung von industriell vorgefertigten Nahrungen, die eine pharmakologische Wirkung im Magen- und Darmbereich entfalten.

Es ist aus EP-A 0 214 362 und JP-A 51 022050 bekannt, daß Huminstoffe im Magen und Darmtrakt eine entzündungshemmende und entgiftende Wirkung haben. Unter den Bezeichnungen Dystikum, Sulumin und Humocarb werden daher Tierarznei- und Fütterungsarzneimittel vertrieben, die ein Gemisch verschiedener natürlicher Huminate mit Molekulargewichten bis zu 100 000 als wirksame Komponente enthalten. Diese Mittel sind bei oraler Aufnahme nicht toxisch und zeigen auch nach langandauernder Verabreichung keine unerwünschten Nebenwirkungen.

Des weiteren entstehen keine toxischen Abbauprodukte und auch die wieder ausgeschiedenen Mittel sind als natürliche Produkte anzusehen. die keine Belastung der Umwelt darstellen.

Es ist aber ein gravierender Nachteil dieser Huminstoffe, daß die Wirkung nur ein Passageeffekt ist, d.h., diese Stoffe wirken nur auf die jeweilige Magen- und Darmschleimhaut, während der entsprechende Nahrungsbrei diese Region passiert. Eine derartige Wirkungsweise ist bei Stoffen, die bei einer Therapie im Humanbereich eingesetzt werden sollen, unerwünscht, denn der Mensch kann nicht ständig essen und auch nicht immer die gleiche Nahrung zu sich nehmen.

Es ist daher Aufgabe der Erfindung, Mittel mit mindestens gleich guter Wirksamkeit im Magen- und Darmbereich wie die bekannten natürlichen Huminstoffe bereitzustellen, die auch hinsichtlich ihrer Toxizität, Nebenwirkungen und Umweltbelastung den natürlichen Huminstoffen entsprechen, deren Wirksamkeitsdauer aber wesentlich verlängert ist, so daß auch nach der Passage des Magen-Darm-Traktes eine entsprechende pharmakologische Wirksamkeit vorhanden ist.

Es ist weiterhin Aufgabe der Erfindung, derartige Mittel im Rahmen industriell vorgefertigter Nahrungsmittel bereitzustellen, die auch für die menschliche Ernährung eingesetzt werden können.

Die Lösung der Aufgabe erfolgt durch Verwendung von synthetisch durch Oxidation mehrwertiger phenolischer Verbindungen hergestellten, wasserlöslichen Alkali- oder Ammoniumhuminate mit einem Molekulargewicht bis zu 50 000 D zur Herstellung von Diätetika gemäß der Ansprüche 1 bis 6, sowie durch Verfahren zu ihrer Herstellung gemäß der Ansprüche 7 und 8.

Es wurde gefunden, daß diese synthetischen, relativ niedermolekularen, wasserlöslichen Alkali- oder Ammoniumhuminate bei oraler Verabreichung ebenfalls im Magen- und Darmbereich eine entzündungshemmende und entgiftende Wirkung haben. Im Gegensatz zu den bislang in Fütterungsarzneimitteln bzw. als Nahrungs- bzw. Futterzusatzstoffen eingesetzten Huminaten werden sie jedoch im Bereich der Darmschleimhaut absorbiert und eingelagert und bewirken dort über einen verlängerten Zeitraum eine etwa gleichbleibende Wirkung, auch wenn der sie enthaltende Nahrungsbrei den Darm längst passiert hat, d. h., diese erfindungsgemäß eingesetzten Huminate zeigen einen deutlichen Retard-Effekt.

Entzündete Darmschleimhaut schwillt schneller ab. Viren und bakterielle Keime werden verstärkt inaktiviert.

Die natürliche Darmflora wird nicht gestört, sondern überraschenderweise stabilisiert. Durch unterschiedliche Ernährung und Klimawechsel, vor allem bei Reisen in entfernte Länder häufig vorkommende Verdauungsstörungen treten bei gleichzeitiger Aufnahme der Huminate nicht auf.

Die synthetisch durch Oxidation mehrwertiger phenolischer Verbindungen hergestellten, wasserlöslichen Alkali- oder Ammoniumhuminate mit einem Molekulargewicht bis zu 50 000 D können daher zur Herstellung von Diätetika verwendet werden und zeigen dabei weitere, teils überraschende Vorteile:

Im Tierversuch zeigen die erfindungsgemäß eingesetzten Huminate einen stark vermifugen Effekt. Nach etwa achttägiger regelmäßiger Verabreichung dieser Huminate sind die behandelten Tiere durchweg wurmfrei. Dies gilt insbesondere bei Zestoden.

Es ist zu erwarten, daß ein derartiger Effekt auch beim Menschen erzielt wird, wenn die Huminate in Form von Diätetika verzehrt werden.

Die Huminate wirken entgiftend. Dies gilt sowohl hinsichtlich eventuell aufgenommener Schwermetalle oder toxischer Substanzen als auch hinsichtlich toxischer Eiweißstoffe, die durch fremde Keime im Darmbereich gebildet werden.

Obwohl die Huminate an der Darmschleimhaut absorbiert werden, werden sie nicht im Körper aufgenommen. D.h. einerseits, daß diese Huminate lokal wirken, andererseits werden Leber und Nieren nicht durch Abbauprozesse oder -produkte belastet, die durch die Huminate bedingt sind.

Ein weiterer praktischer Vorteil der erfindungsgemäß eingesetzten Huminate besteht darin, daß sie vollständig wasserlöslich sind und daher eine Vielzahl von einfacher Zubereitungsmöglichkeiten entsprechender Diätetika erlauben.

Die erfindungsgemäß hergestellten Diätetika enthalten demnach synthetisch durch Oxidation mehrwertiger phenolischer Verbindungen hergestellte, wasserlösliche Alkali- oder Ammoniumhuminate mit einem Molekulargewicht bis zu 50 000 D. Die Huminatmenge kann im weiten Bereich variieren und ist auch davon abhängig, wieviel des Diätetikums jeweils verzehrt wird. Im allgemeinen liegt die Konzentration im Bereich von 0,05 bis 5 Gew.-%.

Sie sollte so eingestellt sein, daß durch den Genuß der erfindungsgemäß hergestellten Diätetika pro kg Körpergewicht pro Tag etwa eine Menge von 0,005 bis 0,5 g Huminat aufgenommen werden.

Im einfachsten Fall liegen die erfindungsgemäß hergestellten Diätetika in flüssiger Form vor, d.h., es sind wäßrige Lösungen der Huminate. 0,05 bis 1 %ige derartige Lösungen, die darüber hinaus Geschmack- bzw. Süßstoffe, Mineral- oder sonstige Aufbaustoffe enthalten können, werden als Getränk eingesetzt.

Erfindungsgemäß hergestellte Diätetika in Form konzentrierterer Lösungen können Speisewürzen zugesetzt sein, sofern die braune Farbe nicht stört und die Würze nicht mehr erhitzt wird.

Analoges gilt auch, wenn die Diätetika in pastöser Form vorliegen, d.h., wenn wäßrige Lösungen der Huminate entweder mit einem Füllstoff ergänzt und/oder mit einem Öl oder Fett emulgiert sind.

Entsprechende Füllstoffe können entweder inerte anorganische Materialien, wie z.B. Silikate oder Schwerspat sein oder natürliches organisches Material, insbesondere cellulosehaltiges Material, wie z.B. Weizennachmehl, Johannisbrotkernmehl oder gemahlene Spelzen.

Die erfindungsgemäß hergestellten Diätetika können auch in fester Form vorliegen, sei es, daß sie mit Huminaten versetzte Rohkostprodukte oder Huminate enthaltende Backwaren sind.

Letztere sind insbesondere kleinteilige Backwaren, wie Biskuit oder Kekse, die sich leicht durch kurzzeitiges Erhitzen backen lassen. Zu ihrer Herstellung werden einer an sich bekannten Teigmasse, wie sie für derartige Produkte üblich ist, 0,1 bis 2 Gew.-% Huminat entweder als Festsubstanz oder als Lösung zugegeben und mit ihr vermischt. Nach der Formgebung wird diese Masse durch kurzzeitiges Erhitzen gebacken.

Es wurde überraschenderweise gefunden, daß durch schnelles Hochfahren der Temperaturen auf 150 bis 180 °C und kurze Backzeiten (bis zu 2 Minuten) eine ausreichende Backwirkung erzielt wird, die Huminate aber dabei nicht merklich zersetzt oder sonstwie geschädigt werden.

Ein besonderes schonendes Backverfahren ist eine Mikrowellenbehandlung, bei der Mikrowellen innerhalb von 10 bis 30 Sekunden mit einer Intensität eingestrahlt werden, daß die geformte Teigmasse dabei gebacken wird.

Erfindungsgemäß einsetzbare und wirksame Huminate sind synthetisch durch Oxidation und dadurch bewirkte Polykondensation von mehrwertigen Phenolen in schwach alkalischem wäßrigem Medium entsprechend EP-A 0 281 678 hergestellte niedermolekulare Ammonium- oder Alkalihuminate. Es sind dunkelbraune, wasserlösliche Produkte mit einem mittleren Molekulargewicht von 1 000, bei einem Streubereich von 250 bis 1 500. Ihre wäßrigen Lösungen zeigen keinen Tyndall-Effekt und fluoreszieren nicht.

Weiterhin wirksam sind aber auch synthetische Huminate mit Molekulargewichten bis zu 50 000 D, sofern sie den folgenden Merkmalen entsprechen:

Sie sind physiologisch positiv wirksam, d. h., sie haben heilende Eigenschaften, sind sehr gering toxisch, embryotoxisch und weder mutagen noch teratogen, bzw. auch nicht cancerogen.

Ihre wäßrigen Lösungen zeigen keinen Tyndall-Effekt und fluoreszieren nicht. Entsprechende Prüfungen dieser Eigenschaften werden zweckmäßigerweise mit wäßrigen Lösungen in einer Konzentration durchgeführt, daß sie noch etwa 50 % der Transmission des eingestrahlten Lichtes haben.

Beispiele für entsprechende, synthetische Huminate sind die gemäß EP-A 0 537 427 hergestellen Produkte.

Ebenso erfindungsgemäß einsetzbar sind modifizierte Ammonium- oder Alkalihuminate, wie sie aus EP-A 0 537 429 bekannt sind. Sie werden hergestellt durch Oxidation der Verbindungen der allgemeinen Formeln 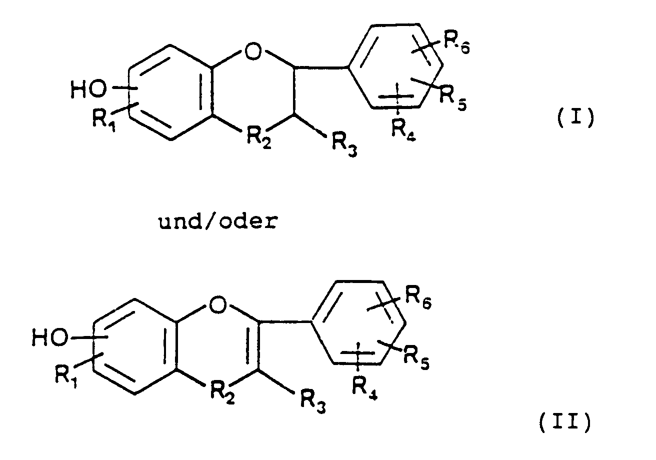 wobei R₁, R₃ und R₄ gleich oder verschieden sind und eine OH-Gruppe oder Wasserstoff darstellt, R₂ eine CO- oder CH₂-Gruppe bedeutet und R₅ und R₆ gleich oder verschieden sind und Wasserstoff eine OH- oder Methoxygruppe, sind, in alkalischer wäßriger Lösung (pH 9).

Dabei können die Verbindungen gemäß der Formeln I und II als einzelne Reinsubstanzen sowie auch im beliebigen Gemisch miteinander eingesetzt werden. Sie können im Reaktionsgemisch auch mit mehrwertigen Phenolen eingesetzt werden, was aus wirtschaftlichen Gründen durchaus angebracht sein kann.

### BEISPIELE

### Beispiel 1

Eine Teigmasse wird hergestellt durch Vermischen von Mehl, Fett, Zucker, Haselnüssen, Eiern, Backpulver, Gewürzen und Wasser.

Ein Teil des Wassers ist eine wäßrige Lösung eines Na-Huminates mit einem durchschnittlichen Molekulargewicht von 1 000 bei einem Streubereich von 300 bis 1 500, hergestellt durch Oxidation von Hydrochinon gemäß dem Beispiel der EP-B 0 281 678.

Die Huminatmenge ist so bemessen, daß die fertige Teigmasse einen Gehalt an Na-Huminat von 1 Gew.-% hat.

Die Teigmasse wird zu Keksen geformt, die in einem Durchlaufofen kurzfristig auf 170 °C erhitzt und 1 Minute auf dieser Temperatur gehalten werden. Die so erhaltenen gebackenen Kekse sind etwas dunkler und süßer als Vergleichskekse aus einer analogen Teigmasse ohne Huminate. Ansonsten bestehen geruchlich und geschmacklich keine Unterschiede.

### Beispiel 2

Die Hälfte der Teilnehmer einer Reisegruppe in ein Land mit vermindertem hygienischem Standard aß 7 Tage lang vor und während der Reise pro Person täglich jeweils 5 Kekse gemäß Beispiel 1.

Diese Reiseteilnehmer hatten während der Reise keine Verdauungsprobleme, während 60 % der restlichen Teilnehmer der Reisegruppe (Kontrollgruppe) nach einigen Tagen an Diarrhoe litten.

## Patentansprüche

1. Verwendung von synthetisch durch Oxidation mehrwertiger phenolischer Verbindungen hergestellten, wasserlöslichen Alkali- oder Ammoniumhuminaten mit einem Molekulargewicht bis zu 50 000 D zur Herstellung von Diätetika.

2. Verwendung nach Anspruch 1, **dadurch gekennzeichnet**, daß die Huminate in gelöster Form vorliegen.

3. Verwendung nach Anspruch 1, **dadurch gekennzeichnet**, daß die Huminate in fester Form vorliegen.

4. Verwendung nach den Ansprüchen 1 und 2, **dadurch gekennzeichnet**, daß die Diätetika Getränke sind.

5. Verwendung nach den Ansprüchen 1 und 3, **dadurch gekennzeichnet**, daß die Diätetika mit Huminaten versetzte Rohkostprodukte sind.

6. Verwendung nach den Ansprüchen 1 und 3, **dadurch gekennzeichnet**, daß die Diätetika Backwaren sind.

7. Verfahren zur Herstellung von Diätetika gemäß Anspruch 6, **dadurch gekennzeichnet**, daß eine Teigmasse hergestellt wird, die 0,1 bis 2 Gew.-% Huminat enthält und daß diese Masse nach Formgebung durch kurzzeitiges Erhitzen gebacken wird.

8. Verfahren nach Anspruch 7, **dadurch gekennzeichnet**, daß die geformte Teigmasse durch Mikrowellenbehandlung innerhalb von 10 bis 30 sec gebacken wird.

## Claims

1. Use of synthetic water-soluble alkali metal or ammonium huminates having a molecular weight of up to 50 000 D produced by oxidation of multivalent phenolic compounds for the manufacturing of dietary products.

2. Use according to claim 1 characterised in that the huminates are present in dissolved form.

3. Use according to claim 1 characterised in that the huminates are present in solid form.

4. Use according to claim 1 characterised in that the dietary products are beverages.

5. Use according to claims 1 and 2 characterised in that the dietary products are raw fruits containing said huminate.

6. Use according to claims 1 and 3 characterised in that the dietary products are baked goods.

7. Process for the production of dietary products according to claim 6 characterised in that a flour dough is formed containing 0,1 to 2 % by weight of said huminate and that the dough is baked by heating for a short period of time after shaping.

8. Process according to claim 7 characterised in that baking of the shaped dough is effected by microwave treating for 10 to 30 seconds.

## Revendications

1. Utilisation d'huminates d'ammonium et d'alcalis, solubles dans l'eau, préparés de manière synthétique par oxydation de composés phénoliques polyvalents, avec un poids moléculaire jusqu'à 50.000 D pour la préparation de produits diététiques.

2. Utilisation selon la revendication 1, caractérisée en ce que les huminates se trouvent sous forme solubilisée.

3. Utilisation selon la revendication 1, caractérisée en ce que les huminates se trouvent sous forme solide.

4. Utilisation selon les revendications 1 et 2, caractérisée en ce que les produits diététiques sont des boissons.

5. Utilisation selon les revendications 1 et 3, caractérisée en ce que les produits diététiques sont des produits à base de crudités, contenant des huminates.

6. Utilisation selon les revendications 1 et 3, caractérisée en ce que les produits diététiques sont des produits de boulangerie.

7. Procédé de préparation de produits diététiques selon la revendication 6, caractérisé en ce qu'on prépare une pâte qui contient 0,1 à 2 % en poids d'huminate et en ce que cette masse est cuite après moulage par un échauffement de courte durée.

8. Procédé selon la revendication 7, caractérisé en ce que la pâte moulée est cuite par un traitement aux micro-ondes en 10 à 30 secondes.
